# EUROPEAN PATENT APPLICATION

(11) **EP 1 464 312 A1**
(43) Date of publication of application: **06.10.2004**
(21) Application number: 03007538.6
(22) Date of filing: 01.04.2003
(51) Int. Cl.: A61F 9/013, A61B 17/32

(54) **Blade for a surgical device for processing the cornea**

(71) Applicant: Carriazo, Cesar C., Dr., Baranquilla (CO)
(72) Inventor: Carriazo, Cesar C., Dr., Baranquilla (CO)
(74) Representative: Hofstetter, Alfons J., Dr.rer.nat.

(57) **Abstract**

The present invention discloses a blade for a surgical device, namely a device for processing the cornea of a human or animal eye, comprising a blade body (32, 32'), a front and a rear edge (12, 12', 12", 12''', 14, 14'), and two lateral edges (16, 16', 18, 18') connecting the front and rear edges (12, 12', 12", 12"', 14, 14') of the blade (10, 10', 40, 40', 50, 50', 60, 60'), the front edge (12, 12', 12", 12'") serving for processing the cornea, wherein an end (30, 30', 30", 30'") of the front edge (12, 12', 12", 12''') of the blade (10, 10', 40, 40', 50, 50', 60, 60') is formed bluntly, such that it is not suitable for severing the cornea. The present invention further relates to a surgical device, namely a device for processing the cornea of a human or animal eye.

## Description

The present invention relates to a blade for a surgical device, namely a device for processing the cornea of a human or animal eye, comprising a blade body, a front and a rear edge, and two lateral edges connecting the front and rear edges of the blade, the front edge serving for processing the cornea. The present invention further relates to a surgical device, namely a device for processing the cornea of a human or animal eye.

Refractive corneal surgery with an excimer laser is presently conducted in one of two ways: Photorefractive keratectomy (PRK) and Laser in situ Keratomileusis (LASIK).

In the PRK technique, the epithelial layer is removed and destroyed manually or with laser, to expose the Bowman layer, so that the laser beam is applied directly to the patient's Bowman layer according to the particular refractive defect. The laser system, thus, destroys an anterior portion of the cornea according to the machine's nomogram, leaving the uncovered stroma. Later, during the healing process, which takes a few days, the stroma will be covered with new epithelial cells. One of the shortcomings of the PRK technique is that the epithelial layer is removed from the eye and the Bowman layer or membrane is destroyed by the direct application of the laser beam to the cornea.

Due to the removal of the epithelium from the eye, the epithelial layer has to regrow over the cornea without the Bowman layer, producing in some cases a corneal opacity known as haze, which decreases the visual acuity of the patient.

Recently, using substances to release the adherence of the epithelial cells to the Bowman layer, this layer can be separated with a blunt spatula; later, after the application of the laser, the Bowman layer has to be reapplied to its original place. This technique is a modified PRK surgery known as LASEK. During the manipulation with these blunt instruments, crease formation takes place, altering the organization of the epithelial cells, destroying many cells, which is going to slow its recovery.

The LASIK technique involves the use of a microkeratome, which makes an access cut across the anterior portion of the cornea. More specifically, the microkeratome makes a corneal lamellar resection to create a corneal flap and hinge. The flap includes corneal tissue from the epithelial, Bowman, and the anterior stromal layers. The corneal flap is typically circular, having a diameter between 7 and 10 mm, and an average of about 160 microns (µm) in thickness. The corneal flap allows for the corneal stroma to be exposed to the ablation by the appropriate laser beam for correcting for the patient's refractive defect. Following the laser application, the corneal flap is returned to its initial position and the recovery is faster than another technique. In this technique, the ablation is not performed at the corneal surface, and the patient suffers no destruction of the Bowman layer.

To carry out LASIK procedures, all known manual, automatic and mechanical microkeratomes use blades to cut the corneal flaps (circular stromal tissue that includes the epithelial layer, Bowman and anterior face of the corneal stroma). All blades used by this microkeratomes have a cutting edge that allows for penetrating and cutting the Bowman layer and the corneal stroma.

Maintaining the corneal structure without risking to weaken it and to induce corneal ecstasies is directly related to the residual final thickness of the stromal bed. The PRK has a lower risk of producing corneal ecstasies than the LASIK, since in the former only the epithelium will be removed, while in the LASIK, the corneal disk has 160 microns on the average. Starting with an average corneal thickness of 500 microns, in PRK, when removing just the epithelium, which has an average thickness of 50 microns, it means 90% of the initial corneal thickness to realize the ablation, while in LASIK, when cutting a corneal disk that has about 150 microns and more on the average, we have only 70% of the initial thickness. In LASIK we can work quietly providing a stromal bed superior to 250 microns which is achieved when low defects are corrected when the cornea is thick (600 microns or more).

With the LASEK technique, it is sought for the advantages of the PRK as much like the LASIK. Since in reapplying the same epithelium from the patient to the original place, the pain is lower than in the PRK, the risk of haze is smaller and the recovery, although it is still slow, is faster than in the PRK.

Therefore, it is one object of the invention to improve the LASEK technique and to be able to create an epithelial flap with minimum manipulation, thereby improving the results of this surgical technique.

Therefore, especially, it is the object of the present invention to provide a blade for a surgical device of the initially mentioned type, which can be used in known surgical devices for processing the cornea of the human or animal eye, wherein the device can be used for performing the so-called LASEK method.

These objects are achieved by a blade according to the features of claim 1 as well as by a surgical device according to the features of claim 11.

Advantageous developments of the inventions are presented in the respective dependent claims.

A blade for a surgical device according to the invention, namely a device for processing the cornea of a human or animal eye has a front edge for processing the cornea, that is the front edge lies towards the eye to be treated, wherein one end of the front edge of the blade is formed bluntly, such that it is not suitable for severing the cornea. Since the end of the front edge of the blade lying towards the eye to be treated is formed bluntly, it is not suitable for cutting the Bowman layer, the stroma or the epithelial layer of the cornea of the human or animal eye. Rather, by the blunt blade, the epithelial layer is lifted from the Bowman layer, without destruction of the epithelial or Bowman layer occurring. Moreover, it is ensured that no changes in the organization of the epithelial cells occur. For lifting the epithelial layer from the Bowman layer, it is necessary to apply one or more alcoholic solutions to the cornea before the severing operation, which weaken or cancel the connection between the epithelial layer and the Bowman layer. Since the epithelial layer is lifted as a whole with a predefined geometry, such as for example as a disk with unitary edges, from the Bowman layer with the aid of the blade, this is advantageously effected without crease formation. Thereby, it is avoided that the epithelial layer is traumatized, which especially cannot be prevented by the manually performed PRK method.

In an advantageous development of the invention, a blade body of the blade has a front and a back side, the front edge of the blade being formed of at least one inclination and a bluntly formed end, and the inclination extending from the front side of the blade body to the back side of the blade body. Such a formation of the blade body and of the front edge of the blade allows for simple and low-cost fabrication of the blade. Further, the blade can advantageously be formed very thin, such that very exactly guiding the blade is possible.

In another advantageous development of the invention, the blade body of the blade has a front and a back side, wherein the front edge of the blade is formed of at least two opposed and converging inclinations and a bluntly formed end, and the inclinations each extend from the front side of the blade body and the back side of the blade body to the end. Such a design of the blade according to the invention advantageously allows for exactly lifting and exactly guiding the blade both in the region of the epithelial layer to be lifted and in the region of the underlying Bowman layer.

In a further advantageous development of the invention, the end of the blade is rounded, straightly or at least partially straightly formed. By virtue of the possibility of the different formation of the blunt front edge of the blade, it is possible to account for different physiological conditions in different patients, especially with respect to the composition of the respective corneal layers.

In a further advantageous development of the invention, the blade body of the blade is provided with a fastener for securing the blade in a blade holder of the device for processing the cornea of a human or animal eye. Thereby, it is possible to use the blade according to the invention in a usual and known microkeratome. Therein, the type of the fastener orients to the design of the blade holder of the employed microkeratome.

In a further advantageous development of the blade according to the invention, it is made of metal, a metal alloy, of plastics or ceramics. However, it is also possible that the front edge of the blade is coated with a material different from the material of the blade. Thereby, it is also ensured that different physiological conditions can be accommodated. Therein, all of the used materials are bio-stable and/or biocompatible.

In further advantageous developments of the invention, the blade and the blade body, respectively, are at least partially formed as a regular or non-regular rectangle. Therein, the front edge of the blade can be formed straightly, partly straight or curved. Also the blade itself can be formed straightly or bent in the horizontal. By virtue of the very different design possibilities of the blade, in turn, it is possible to employ them in all known and already used microkeratomes.

A surgical device according to the invention, namely a device for processing the cornea of a human or animal eye, has a blade according to the invention described above. Therein, the blade can be detachably attached in a blade holder of the device, especially a microkeratome. However, it is also possible to form the blade holder and the blade integrally.

In an advantageous development of the surgical device, it has a suction ring for fitting onto the eye and for partially sucking the cornea of the eye, wherein the blade holder with the blade moves across the suction ring and the blunt end of the front edge of the blade penetrates an epithelial layer of the cornea above the Bowman layer, such that an approximately round epithelial flap arises and is lifted, and the epithelial flap is completely or only partially severed from the remaining epithelial layer or the Bowman layer. With such a surgical device, it is advantageously possible to perform an automated LASEK method without having to accept the disadvantages of the known PRK and LASIK methods. Thus, the surgical device according to the invention allows for lifting the epithelial layer of the cornea from the Bowman layer of the cornea, without excessive trauma of the tissue occurring. Thus, in turn, a fast healing process is ensured.

Further details and features of the present intentions result from the embodiments described below and shown in the drawings. There show:
- Figures 1a to 1c:: a schematic representation of the inventive blade according to a first embodiment;
- Figures 2a to 2c:: a schematic representation of an inventive blade according to a second embodiment;
- Figures 3a to 3c:: a schematic representation of an inventive blade according to a third embodiment;
- Figures 4a to 4c:: a schematic representation of an inventive blade according to a fourth embodiment;
- Figures 5a to 5c:: a schematic representation of an inventive blade according to a fifth embodiment;
- Figures 6a to 6c:: a schematic representation of an inventive blade according to a sixth embodiment;
- Figures 7a to 7c:: a schematic representation of an inventive blade according to a seventh embodiment;
- Figures 8a to 8c:: a schematic representation of an inventive blade according to an eighth embodiment;
- Figure 9:: a schematic representation of a cut course of a microkeratome with a cutting blade according to the prior art;
- Figures 10a to 10c:: a schematic representation of a guidance of a surgical device according to the invention with a blunt blade according to the invention.

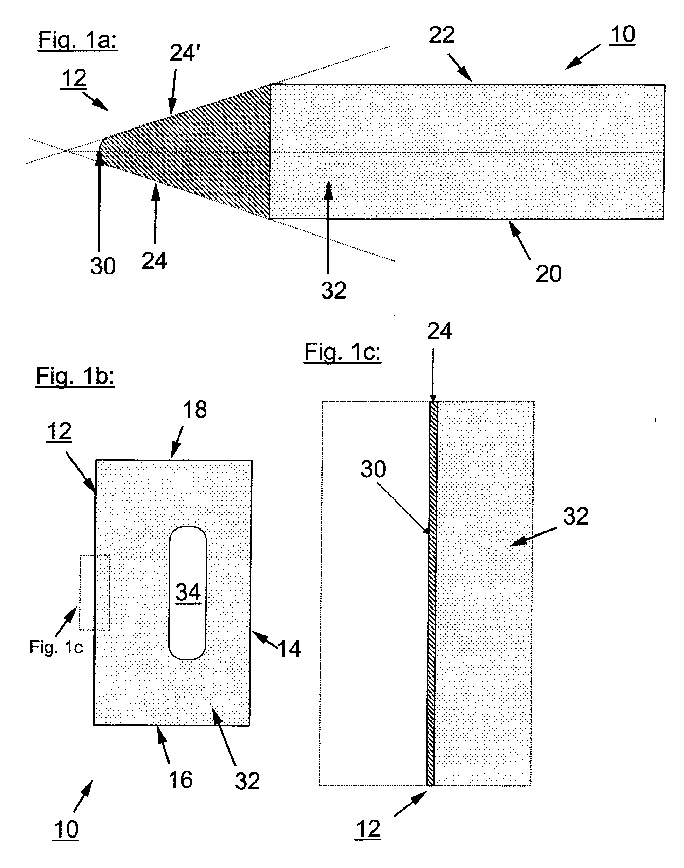

Figures 1 a to 1 c show a blade 10 for a surgical device, namely a device for processing the cornea of a human or animal eye according to a first embodiment. Therein, the blade 10 has a blade body 32, a front and a rear edge 12, 14 and two lateral edges 16, 18 connecting the front and rear edges 12, 14 of the blade 10 (compare to figure 1b). Therein, the front edge 12 serves for processing the cornea and thus lies towards the cornea of the eye to be treated. Further, it is recognized that one end 30 of the front edge 12 of the blade 10 is formed bluntly, such that it is not suitable for severing the cornea, especially the Bowman layer of the cornea. In the illustrated embodiment, the end 30 is formed rounded.

Additionally, the blade body 32 of the blade 10 has a front and a back side 20, 22. Therein, the front edge 12 of the blade 10 is formed of two opposing and converging inclinations 24, 24' and the bluntly formed end 30. It is recognized that the inclinations 24, 24' each extend from the front side 20 of the blade body 32 and the back side 22 of the blade body 32 to the end 30.

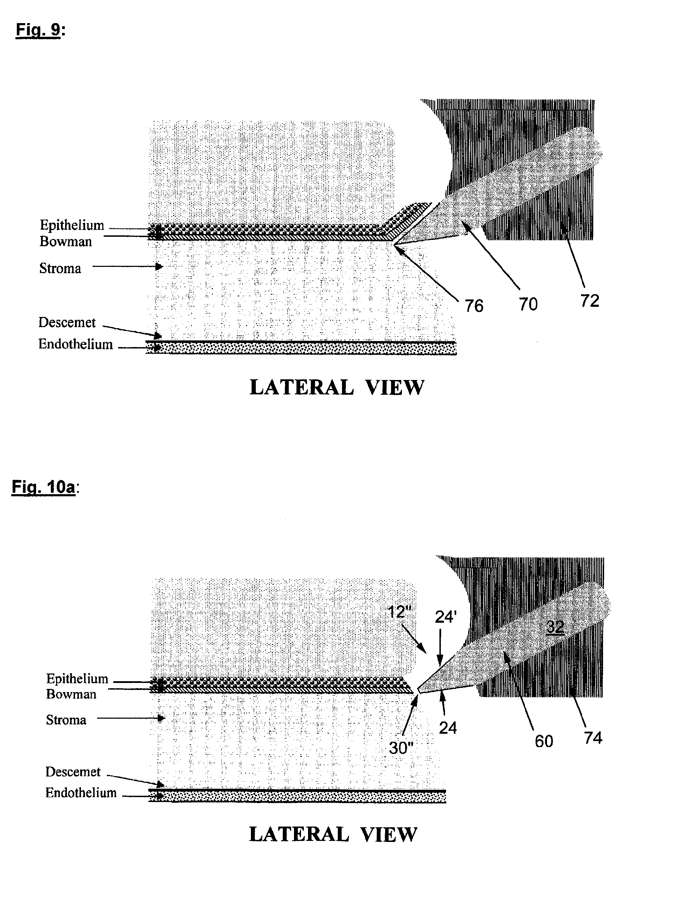

From figure 1b, it becomes clear that the blade body 32 has a fastener 34 for securing the blade 10 in a blade holder 74 (compare to figure 10a) of the device for processing the cornea of a human or animal eye. Therein, the fastener 34 is formed as an opening, into which a corresponding projection of the blade holder 74 can penetrate and secures the blade 10 to the blade holder 74. However, other securing types are also possible. For example, clamping the blade 10 into corresponding lateral guides of a blade holder is conceivable. However, besides the possibilities of detachably attaching the blade 10 to the blade holder, it is also possible that the blade 10 and the blade holder are formed integrally. Incidentally, the illustrated securing possibilities of the blade 10 to corresponding blade holders 74 of the surgical device, especially of a microkeratome, apply also to the embodiments of the blade described below.

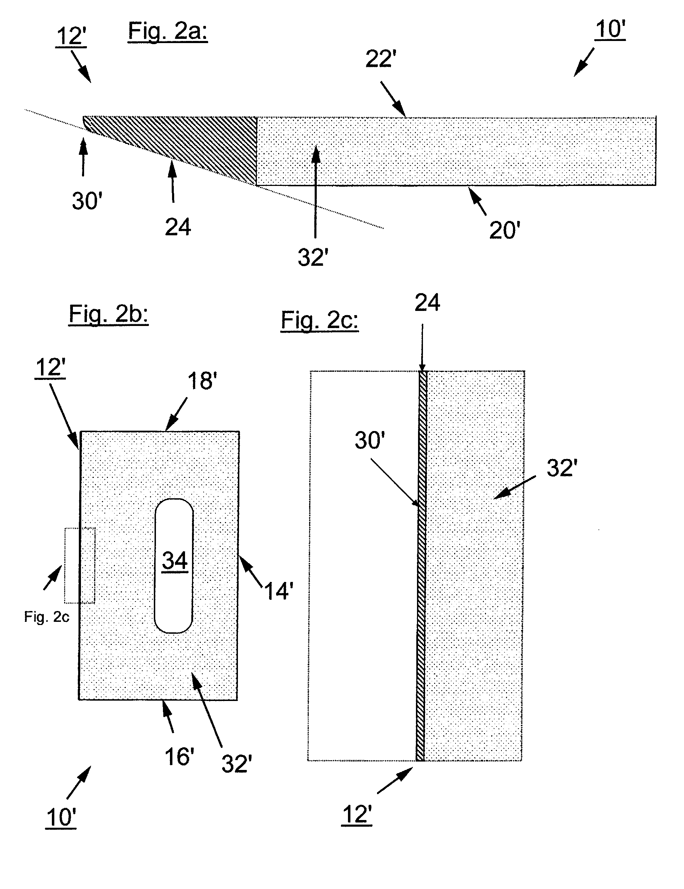

Figures 2a to 2c show a schematic representation of a blade 10' according to a second embodiment. It is recognized that the blade 10' has a blade body 32', wherein the blade body 32' has a front and a back side 20', 22'. Therein, the front edge 12' of the blade 10' is formed of an inclination 24 and a bluntly formed end 30'. Therein, the inclination 24 extends from the front side 20' of the blade body 32' to the back side 22' of the blade body 32'. Therein, the blade 10 has lateral edges 16', 18' connecting the front edge 12' to a rear edge 14' of the blade 10'. A fastener 34 is disposed within the blade 10'.

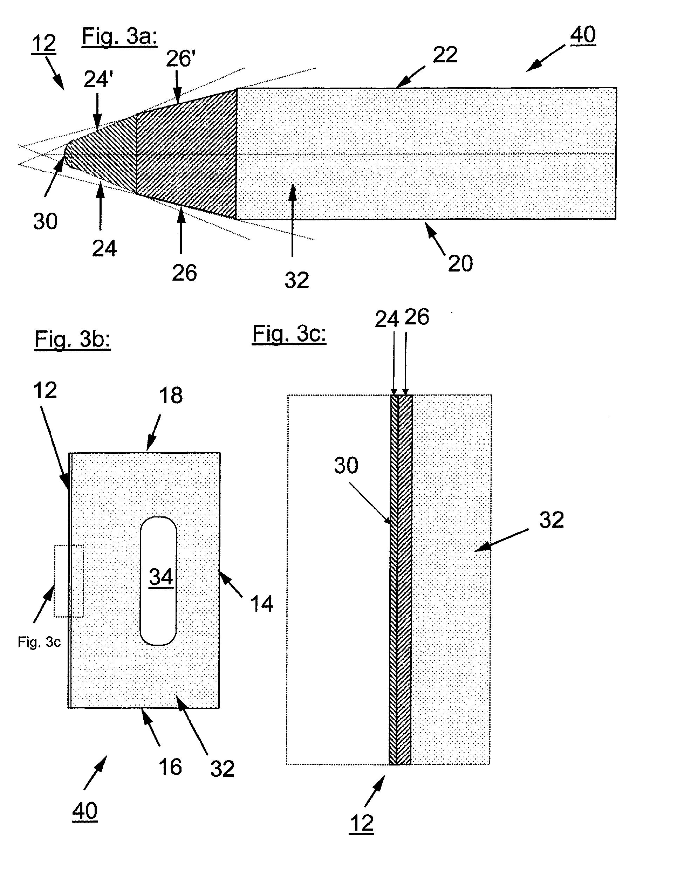

Figures 3a to 3c show a schematic representation of a blade 40 according to a third embodiment. It is recognized that the blade 40 has a blade body 32 with a front and a back side 20, 22. Therein, the front edge 12 of the blade 40 has totally four inclinations 24, 24', 26, 26', wherein the inclinations 24 and 24' as well as 26 and 26' are each formed opposing and each extend to the rounded end 30 of the blade 40.

Further, the blade 40 has lateral edges 16, 18 as well as an end 14 opposing the front end 12. A fastener 34 is disposed within the blade 40. From figure 3c, in the top view, the arrangement of the two inclinations 24, 26 of the end 12 of the blade 40 becomes clear.

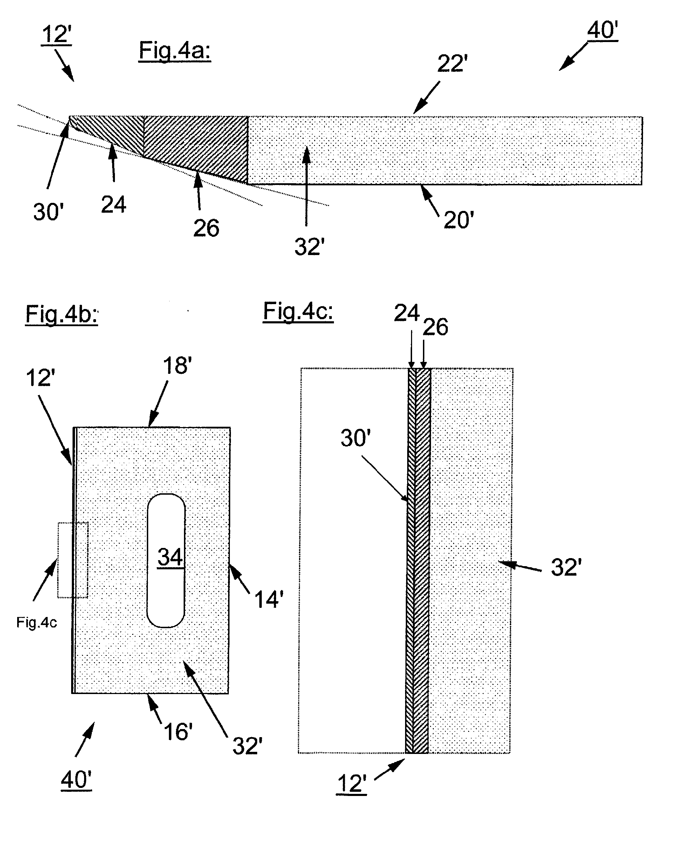

Figures 4a to 4c show a schematic representation of a blade 40' according to a fourth embodiment. It is recognized that the blade 40' has a blade body 32', wherein the blade body 32' has a front and a back side 20', 22'. Therein, the front edge 12' of the blade 40' is formed of two inclinations 24, 26 and a bluntly formed and rounded end 30'. Therein, the inclinations 24, 26 generally extend from the front side 20' of the blade body 32' towards the end 30' or to the back side 22' of the blade body 32', respectively. Further, it is recognized that the blade 40' has lateral edges 16', 18' connecting the front edge 12' to a rear edge 14' of the blade 10'. From figure 4c, in the top view, the arrangement of the inclinations 24, 26 in the region of the front edge 12' becomes clear.

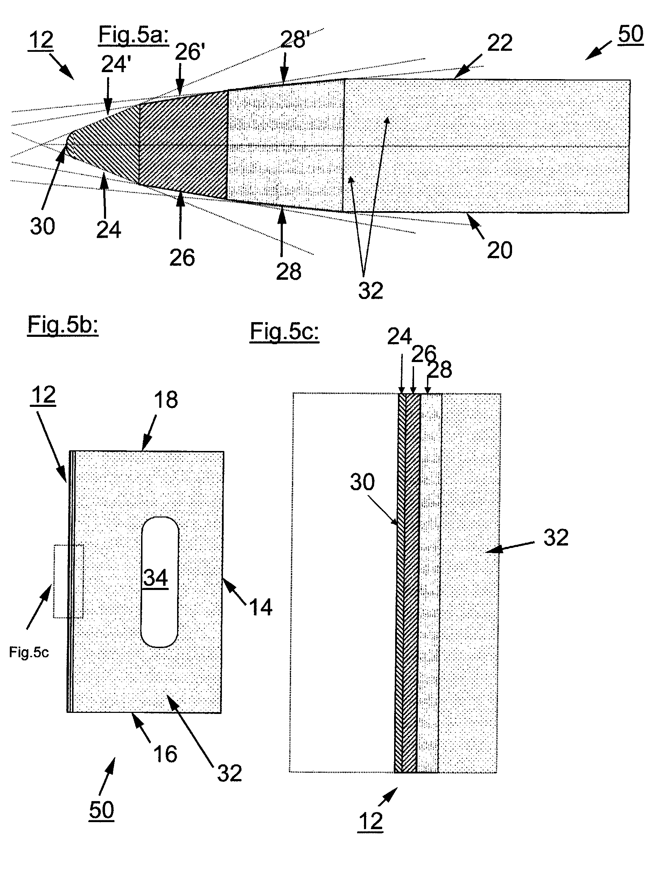

Figures 5a to 5c show a schematic representation of a blade 50 according to a fifth embodiment. Therein, the blade 50 has a blade body 32 with a front and a back side 20, 22. Therein, a front edge 12 of the blade 50 is formed of six inclinations 24, 24', 26, 26', 28, 28' and a bluntly formed and rounded end 30. Therein, the inclinations 24 and 24', the inclinations 26 and 26' as well as the inclinations 28 and 28', respectively, are opposed. It becomes clear, that the inclinations 24, 24', 26, 26', 28, 28' converge towards the rounded end 30 of the blade 50. From figure 5b, it becomes clear, that the blade 50 has lateral edges 16, 18 connecting the front edge 12 to an opposed rear edge 14. The blade body 32 has a fastener 34. From figure 5c, in the top view, the arrangement of the inclinations 24, 26, 28 in the region of the front edge 12 becomes clear.

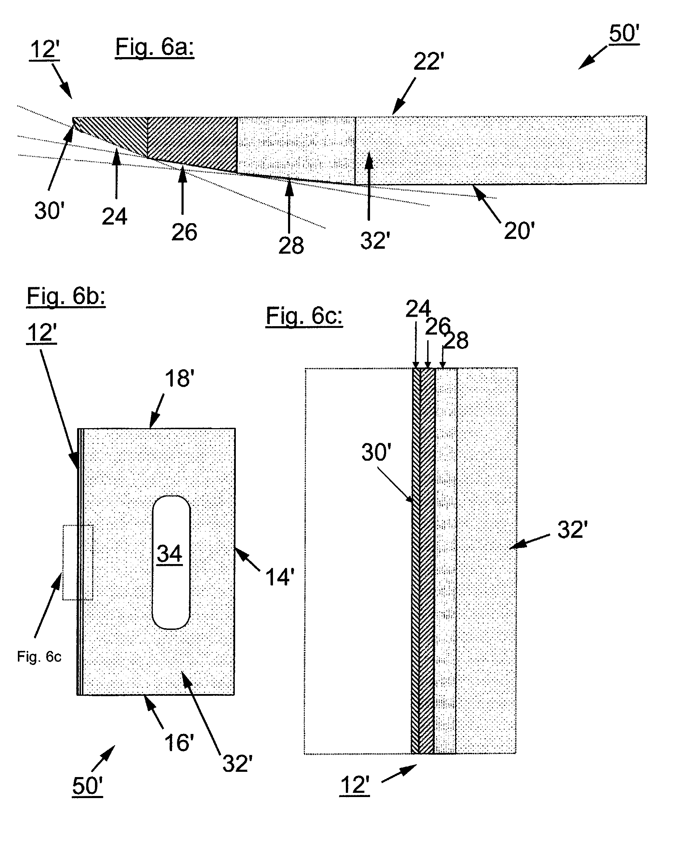

Figures 6a to 6c show a schematic representation of a blade 50' according to a sixth embodiment. It is recognized that the blade 50' has a blade body 32', wherein the blade body 32' has a front and a back side 20', 22'. Therein, the front edge 12' of the blade 50' is formed of totally three inclinations 24, 26, 28 and a bluntly formed end 30'. Therein, the inclinations 24, 26, 28 extend towards the end 30' or from the front side 20' of the blade body 32' to the back side 22' of the blade body 32', respectively. Further, the blade 50' has lateral edges 16', 18' connecting the front edge 12' to the rear edge 14' of the blade 10'. A fastener 34' is formed in the blade body 32'. From figure 6c, in the top view, the arrangement of the inclinations 24, 26, 28 in the region of the front edge 12' becomes clear.

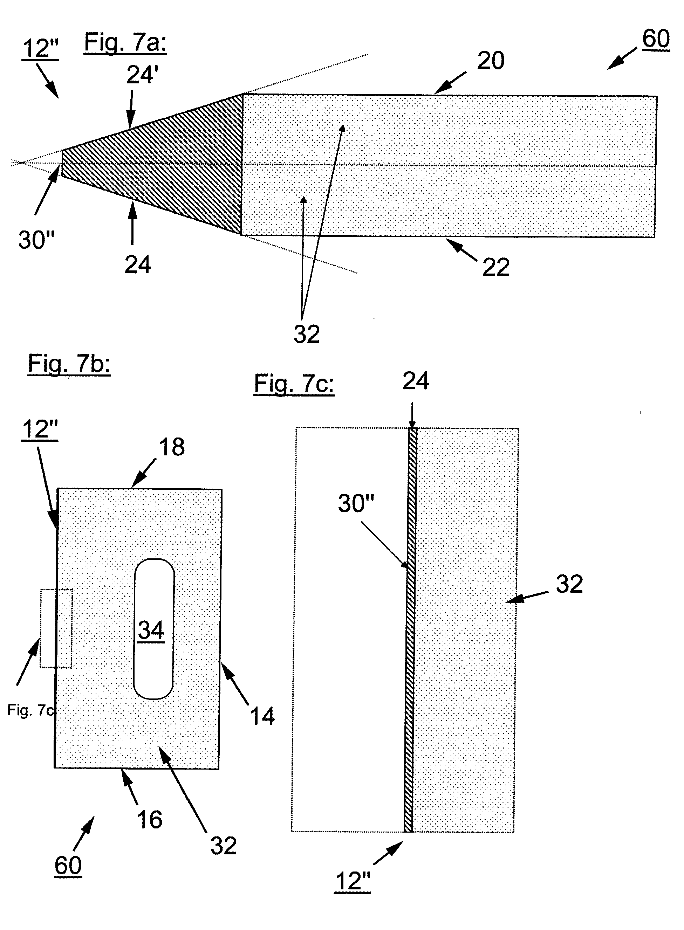

Figures 7a to 7c show a schematic representation of a blade 60 according to a seventh embodiment. Therein, the blade 60 has a blade body 32, a front and a rear edge 12", 14 and two lateral edges 16, 18 connecting the front and the rear edge 12", 14 of the blade 60 (compare to figure 7b). Therein, the front edge 12" serves for processing the cornea and thus lies towards the eye or the cornea of the eye, respectively. Further, it is recognized that one end 30" of the front edge 12" of the blade 60 is formed bluntly. Therein, in the illustrated embodiment, the end 30" is formed straightly. However, it is also possible to form the blunt end 30" only partially straightly. Also a combination of a rounded and a partially straight end is possible. However, it is to be taken care that no cutting edge arises.

Additionally, the blade body 32 of the blade 60 has a front and a back side 20, 22. Therein, the front edge 12" of the blade 60 is formed of two opposed and converging inclinations 24, 24' and the bluntly formed end 30". It is recognized that the inclinations 24, 24' each extend from the front side 20 of the blade body 32 and the back side 22 of the blade body 32 to the end 30".

From figure 6b, it becomes clear, that the blade body 32 has a fastener 34 for securing the blade 60 in a blade holder 74 (compare to figure 10a) of a device for processing the cornea of a human or animal eye. Further, from figure 7b it is recognized that the blade 60 has lateral edges 16, 18 connecting the front edge 12" to the rear edge 14. From figure 7c, in the top view, the arrangement of the inclination 24 in the region of the front edge 12" becomes clear.

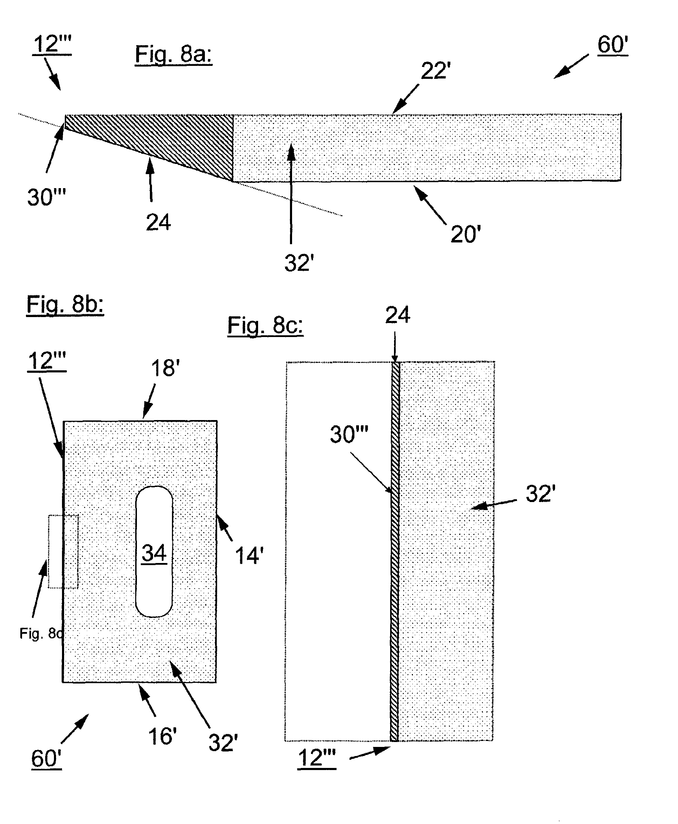

Figures 8a to 8c show a schematic representation of a blade 60' according to an eighth embodiment. It is recognized that the blade 60' has a blade body 32', wherein the blade body 32' has a front and a back side 20', 22'. Therein, a front edge 12"' of the blade 60' is formed of an inclination 24 and a bluntly formed end 30"'. Therein, the inclination 24 extends from the front side 20' of the blade body 32' to the back side 22' of the blade body 32' or towards the bluntly formed end 30"', respectively. Further, it is recognized that the blade 60' has lateral edges 16', 18' connecting the front edge 12'" to the rear edge 14' of the blade 60'. A fastener 34 is formed in the blade body 32'. From figure 8c, in the top view, the arrangement of the inclination 24 in the region of the front edge 12'" is recognized.

Figure 9 shows a schematic representation of a cutting course of a known microkeratome with a usual cutting blade 70. Therein, the blade 70 is fixed in a blade holder 72 of the known microkeratome. It is recognized that the blade 70 has a cutting edge 76. Further, it is recognized that the blade 70 known from the prior art penetrates the stroma of the cornea of the eye with its cutting blade 76, and thus removes a relatively thick region of the cornea. As already initially described in detail, the latter is very disadvantageous in performing corrections for defects of sight of the eye.

In figures 10a to 10c, the guidance of a surgical device with a blunt blade 60 is shown schematically. The following designs are exemplarily illustrated using the blade 60, that is according to the seventh embodiment of the invention. However, the designs also apply correspondingly to all of the other embodiments of the blade according to the invention.

From figure 10a, it is recognized that the blade 60 is secured in a blade holder 74 of a surgical device (not shown) such as a microkeratome. Therein, the blade 60 has a blade body 32 as well as the opposing inclinations 24, 24' converging towards the end 30" of the front edge 12" of the blade 60. It is recognized that the blunt end 30" of the blade 60 is applied in the boundary region between the epithelial layer and the Bowman layer.

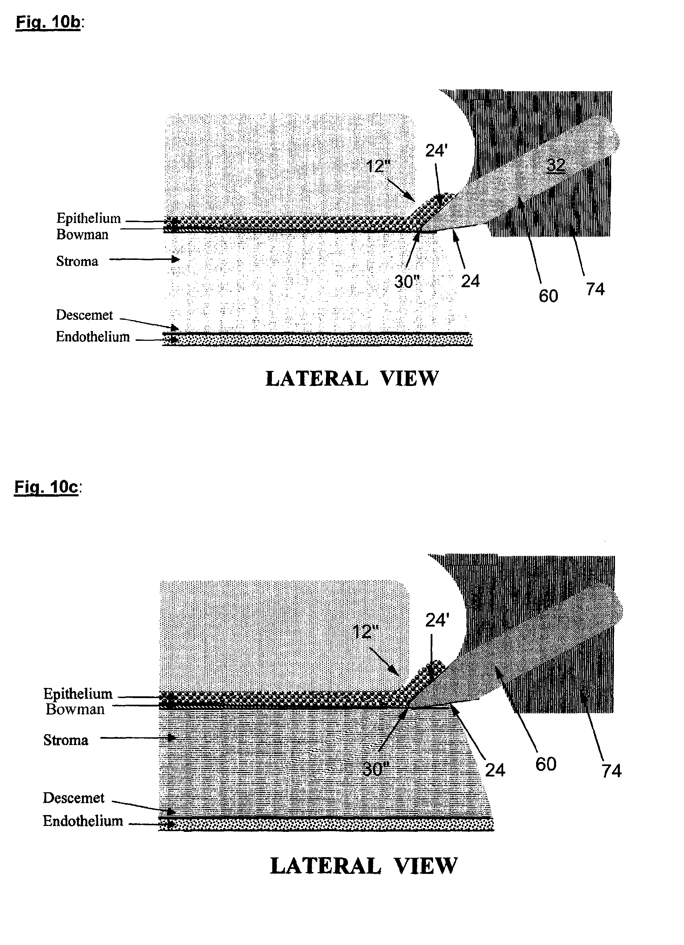

From figure 10b, it becomes clear that the blunt edge 60 or the blunt end 30" thereof lifts the epithelial layer from the Bowman layer. Therein, the epithelial layer has previously been besprinkled with an alcoholic solution, which allows for releasing or lifting the epithelial layer from the Bowman layer, respectively. From figures 10b and 10c, it becomes clear, that lifting the epithelial layer from the Bowman layer is effected without destroying the mentioned layers. Complete or partial release of or completely or partially lifting the epithelial layer from the Bowman layer, respectively, is effected. Penetration of the blade 60 into other layers of the cornea such as the stroma is not possible. Since exclusively the epithelial layer is removed or lifted, respectively, the arising flap is relatively thin. Since both the epithelial layer, especially the lifted region of the epithelial layer, and the Bowman layer are not traumatized, a fast healing process after performing the correction for defects of sight of the eye is ensured. Additionally, the severed portion of the epithelial layer can be reapplied to the corresponding regions of the cornea straight away and especially without crease formation or the like.

The described blades 10, 10', 40, 40', 50, 50', 60, 60' can be formed of metal, a metal alloy, of plastics or ceramics. Therein, the front edges 12', 12", 12'" of the blades 10, 10', 40, 40', 50, 50', 60, 60' can be coated with a material different from the material of the blade 10, 10', 40, 40', 50, 50', 60, 60'. Moreover, it is possible that the blades 10, 10', 40, 40', 50, 50', 60, 60' are formed at least partially as regular or non-regular rectangles. Further, the blade bodies 32, 32' can be formed straightly or bent. The front edges 12, 12', 12", 12"' of the blades 10, 10', 40, 40', 50, 50', 60, 60' can be formed straightly or curved.

A surgical device, namely a device for processing the cornea of a human or animal eye, has a blade 10, 10', 40, 40', 50, 50', 60, 60'. Therein, the device (not shown) can have a suction ring for fitting onto the eye and for partially sucking the cornea of the eye. Therein, a blade holder 74 with the blade 10, 10', 40, 40', 50, 50', 60, 60' is moved across the suction ring, wherein a blunt end 30, 30', 30", 30"' of the front edge 12, 12', 12", 12"' of the blade 10, 10', 40, 40', 50, 50', 60, 60' penetrates an epithelial layer of the cornea above the Bowman layer such that an approximately round epithelial flap arises and the epithelial flap is completely or only partially severed from the remaining epithelial layer. The blunt blade 10, 10', 40, 40', 50, 50', 60, 60' according to the invention is formed such that it can be employed in nearly all known microkeratomes for performing the LASEK method.

## Claims

1. Blade for a surgical device, namely a device for processing the cornea of a human or animal eye, comprising a blade body (32, 32'), a front and a rear edge (12, 12', 12", 12"', 14, 14'), and two lateral edges (16, 16', 18, 18') connecting the front and rear edges (12, 12', 12", 12"', 14, 14') of the blade (10, 10', 40, 40', 50, 50', 60, 60'), the front edge (12, 12', 12", 12"') serving for processing the cornea,
**characterized in that**
an end (30, 30', 30", 30"') of the front edge (12, 12', 12", 12"') of the blade (10, 10', 40, 40', 50, 50', 60, 60') is formed bluntly, such that it is not suitable for severing the cornea.

2. Blade according to claim 1,
**characterized in that**
the blade body (32') has front and back sides (20', 22'), wherein the front edge (12', 12"') of the blade (10', 40', 50', 60') is formed of at least one inclination (24, 26, 28) and a bluntly formed end (30', 30"'), and the inclination (24, 26, 28) extends from the front side (20') of the blade body (32') to the back side (22') of the blade body (32').

3. Blade according to claim 1,
**characterized in that**
the blade body (32) has front and back sides (20, 22), wherein the front edge (12, 12") of the blade (10, 40, 50, 60) is formed of at least two inclinations (24, 26, 28, 24', 26', 28') and a bluntly formed end (30, 30"), and the inclinations (24, 26, 28, 24', 26', 28') each extend from the front side (20) of the blade body (32) and the back side (22) of the blade body (32) to the end (30, 30").

4. Blade according to any one of claims 2 or 3,
**characterized in that**
the end (30, 30', 30", 30"') of the blade (10, 10', 40, 40', 50, 50', 60, 60') is formed rounded or straightly or partly straight.

5. Blade according to any one of the preceding claims,
**characterized in that**
the blade body (32, 32') has a fastener (34) for securing the blade (10, 10', 40, 40', 50, 50', 60, 60') in a blade holder (74) of the device for processing the cornea of a human or animal eye.

6. Blade according to any one of the preceding claims,
**characterized in that**
the blade (10, 10', 40, 40', 50, 50', 60, 60') consists of metal, a metal alloy, of plastics or ceramics.

7. Blade according to claim 6,
**characterized in that**
the front edge (12, 12', 12", 12"') of the blade (10, 10', 40, 40', 50, 50', 60, 60') is coated with a material different from the material of the blade (10, 10', 40, 40', 50, 50', 60, 60').

8. Blade according to any one of the preceding claims,
**characterized in that**
the blade (10, 10', 40, 40', 50, 50', 60, 60') is at least partially formed as a regular or non-regular quadrangle.

9. Blade according to claim 8,
**characterized in that**
the front edge (12, 12', 12", 12"') of the blade (10, 10', 40, 40', 50, 50', 60, 60') is formed straightly or curved.

10. Blade according to any one of the preceding claims,
**characterized in that**
the blade (10, 10', 40, 40', 50, 50', 60, 60') is formed straightly or bent.

11. Surgical device, namely a device for processing the cornea of a human or animal eye, comprising a blade (10, 10', 40, 40', 50, 50', 60, 60') according to any one of the preceding claims.

12. Surgical device according to claim 11,
**characterized in that**
the blade (10, 10', 40, 40', 50, 50', 60, 60') is detachably attached in a blade holder (74) of the device.

13. Surgical device according to claim 11,
**characterized in that**
the blade (10, 10', 40, 40', 50, 50', 60, 60') is integrally formed with a blade holder (74) of the device.

14. Surgical device according to claim 12 or 13,
**characterized in that**
the device has a suction ring for fitting onto the eye and partially sucking the cornea of the eye, and the blade holder (74) with the blade (10, 10', 40, 40', 50, 50', 60, 60') moves across the suction ring, wherein the blunt end (30, 30', 30", 30"') of the front edge (12, 12', 12", 12"') of the blade (10, 10', 40, 40', 50, 50', 60, 60') penetrates an epithelial layer of the cornea over the Bowman layer, such that an approximately round epithelial flap arises and the epithelial flap is completely or only partially severed from the remaining epithelial layer.
